# EUROPEAN PATENT APPLICATION

(11) **EP 2 781 214 A1**
(43) Date of publication of application: **24.09.2014**
(21) Application number: 13382106.6
(22) Date of filing: 22.03.2013
(51) Int. Cl.: A61K 9/20, A61K 31/519

(54) **Formulation of amorphous calcium L-5-methyltetrahydrofolate (L-5-MTHF-Ca)**

(71) Applicant: Chemo Research, S.L., 28050 Madrid (ES)
(72) Inventor: Hernandez Herrero, Gonzalo, 28050 Madrid (ES); Ronchi, Celestino, 20124 Milano (IT); Díaz Del Consuelo, Isabel, 28500 Arganda Del Rey (ES)
(74) Representative: Oficina Ponti, SLP

(57) **Abstract**

The present invention relates to a dosage form which comprises amorphous calcium L-5-methylfolate and cysteine as stabilizing agent and optionally, further auxiliary agents.

The dosage forms according to the invention resolves the problem of unstability of amorphous calcium L-5-methylfolate to the oxidation by air.

## Description

### Field of the invention

The present invention relates to a stabilized mixture of amorphous calcium L-5-methyltetrahydrofolate and cysteine and to the use of said stabilized mixture in the production of a dosage form.

### Background of the invention

Calcium L-5-methyltetrahydrofolate is the Nonproprietary Name of N-{4-[[((6S)-2-amino-1,4,5,6,7,8-hexahydro-5-methyl-4-oxo-6-pteridinyl)methyl]amino]benzoyl}-L-glutamic acid, calcium salt.

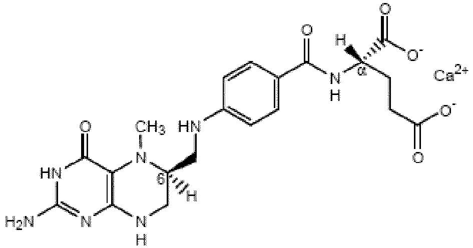

Calcium L-5-methyltetrahydrofolate is commercialized by Merk Eprova under the trademark METAFOLIN^{®} for use in the manufacture of foods for particular nutritional uses.

Calcium L-5-methyltetrahydrofolate is predominantly used in nutritional formulations for pregnant women indicated for the prevention of neural tube defects of the fetus. Is also used for the treatment of high risk recurrent pregnancy loss; risk of anemia due to impaired folic acid absorption; for the treatment of early memory loss, mild to moderate cognitive impairment and vascular dementia or Alzheimer disease.

Additionally, calcium L-5-methylfolate has been used as component of contraceptive compositions comprising a progestogen such as drospirenone and a estrogen such as ethinyl estradiol indicated for raise folate levels in women who choose to use an oral contraceptive for contraception for the purpose of reducing the risk of a neural tube defect and other congenital malformations caused by folate deficiency, in a pregnancy conceived while taking the product or shortly after discontinuing the product.

L-5-methylfolate is the predominant natural form of folates in many foods. It is also the essential form in which folates occur and are stored in the human body. In some foods (e.g. orange juice) it is the only folate present.

During absorption, all natural folates are converted to L-methylfolate which is the only form of folate to enter the human circulation. Unlike folic acid, L-5-methylfolate has to be converted to tetrahydrofolate (THF) via the vitamin B-12-dependent enzyme methionine synthase before it can participate in other folate-dependent reactions. When vitamin B-12 is deficient, L-5-methylfolate is not converted to tetrahydrofolate and thus is not able to ameliorate megaloblastic anemia. For that reason, L-5-methylfolate is the only reduced form of folate that does not mask vitamin B-12 deficiency.

Main problem associated with the pharmaceutical use of calcium L-5-methylfolate is that it is extremely unstable, and it is highly susceptible to oxidation by air, and it is therefore difficult to formulate as a pharmaceutical active ingredient of food additive.

Patent US 6,441,168 solves this problem by providing crystalline forms I, II, III and IV of calcium L-5-methylfolate. These crystalline forms are stable at room temperature, practically without limitation and are therefore suitable for the production of pharmaceutical compositions or food additives.

Main problem associated with the use of crystalline forms of a compound in the production of dosage forms is the inter-conversion of the crystalline forms during the process for its production. It is well known that the physical stability of polymorphs, in particular of hydrates and anhydrous forms may depend upon the relative humidity and/or temperature of the environment, and the most stable form may switch as the humidity/temperature is varied. Transitions between crystalline forms can occur and thus affect the dissolution rate and perhaps bioavailability of the drug in the dosage form.

For example, patent US 6,441,168 teaches that crystalline form II of calcium L-5-methylfolate can be converted into form I by adding water to the crystals, e.g. by treatment with water in a humidity cabinet at 90°C. Additionally, form I of calcium L-5-methylfolate can be converted into forms II, III or IV by drying under vacuum at 70°C (conversion into form II), or by thermal treatment at a temperature above 90 °C (conversion into form III) or above 95°C (conversion into form IV).

Thus, in order to avoid the conversion of the crystalline form during the production process, careful control of the conditions used (humidity, temperature) should be made to avoid the inter-conversion between polymorphic forms.

Furthermore, patent application US 2008160004 teaches that Metafolin (commercialized crystalline form of calcium L-5-methylfolate) is degraded during granulation with the other components of the composition. Further, only 60% of Metafolin remains in the compositions obtained by granulation after a storage time of one month at 40°C. Dry admixture of Metafolin after the completion of the granulation process is necessary to stabilize Metafolin in the formulations disclosed.

The alternative to the crystalline forms of calcium L-5-methylfolate, i.e. the amorphous form of calcium L-5-methylfolate can be obtained according to the process disclosed in patent application US 2010168117. However, in the presence of oxygen, amorphous calcium L-5-methylfolate is very unstable and therefore, it is considered as less suitable for the production of dosage forms.

Thus, problems in respect of the physical and chemical stability of calcium L-5-methylfolate are not yet satisfactorily solved. There is still a need for improving the stability of calcium L-5-methylfolate, by sufficiently suppressing decomposition and conversion between crystalline forms when it is formulated into an oral dosage form, and during the storage time.

### Summary of the invention

The dosage form of the invention resolves the aforesaid disadvantages, presenting other advantages that will be described.

Present inventors have surprisingly found that amorphous calcium L-5-methylfolate may be stabilized by mixing it with cysteine. Said mixture can be processed into a dosage form, thus providing stabilized preparations comprising amorphous calcium L-5-methylfolate.

Thus, the object of this invention is to provide stabilized dosage forms of amorphous calcium L-5-methylfolate.

Thus, an aspect of the present invention refers to a dosage form which comprises amorphous calcium L-5-methylfolate and cysteine as stabilizing agent and optionally, further auxiliary agents.

Another aspect of the present invention refers to a process for the production of a dosage form comprising amorphous calcium L-5-methylfolate by mixing amorphous calcium L-5-methylfolate with cysteine and then processing the mixture to produce a stable dosage form.

Another aspect of the invention is the use of the dosage form of the invention for the production of a pharmaceutical composition, for example, of contraceptive compositions or for the production of nutraceutical compositions.

Another aspect of the invention is the use of cysteine as stabilizing agent for the stabilization of amorphous L-5-methylfolate.

### Detailed description of the invention

All terms as used herein in this application, unless otherwise stated, shall be understood in their ordinary meaning as known in the art. Other more specific definitions for certain terms as used in the present application are set forth below and are intended to apply uniformly through-out the specification and claims unless otherwise expressly set out definition provides a broader definition.

After numerous assays, the present inventors have found that cysteine is capable of stabilizing amorphous calcium L-5-methylfolate. Additionally, the present inventors have surprisingly found that the amorphous form of calcium L-5-methylfolate is stable maintained in the dosage forms containing the mixture of L-5-methylfolate and cysteine.

Present inventors have tested the stabilization effect on calcium L-5-methylfolate of several reducing agents and antioxidants, such as methionine, sodium ascorbate, calcium ascorbate, ascorbyl palmitate, cytric acid, trisodium dihydrated citrate, α-tocopherol (vitamin E), propyl gallate, lipoic acid, buthylhydroxytoluene (BHT), butylhydroxyanisol (BHA), butylhydroxytoluene (BHT), polacrilin potassium and mixtures thereof.

None of the agents listed above provide a satisfactorily practical stabilizing effect of amorphous calcium L-5-methylfolate compared as the cysteine one, as it can be see in Tables 1 to 6 included below on the Example section, specifically at the end of example 6.

The present inventors have surprisingly found that is possible to stabilize amorphous calcium L-5-methylfolate by mixing it with cysteine, as it is shown in Tables 1 to 6 (see example 6). Furthermore, said mixture can be processed into a stable dosage form, thus providing stabilized formulations comprising amorphous calcium L-5-methylfolate. The incorporation of cysteine as stabilizing agent into the dosage form of the invention allows stabilization of calcium L-5-methylfolate during the blending, granulation, spray-drying or compression process. Additionally, the amorphous form of calcium L-5-methylfolate is maintained stable in the final dosage form.

Thus, the present invention relates to a dosage form comprising effective amount of amorphous calcium L-5-methylfolate as active ingredient, cysteine as stabilizing agent and optionally further auxiliary agents.

As used herein, the term "dosage form" applies to any solid or semi-solid composition designed to contain a specific pre-determined amount or dose of a certain ingredient, for example an active ingredient as defined above.

The active substance of the dosage form of this invention is the amorphous calcium L-5-methylfolate, which may be prepared, for instance, in accordance to the process disclosed in patent application US 2010168117, as a general process on pages 1 to 2, paragraphs [0027]-[0034], and specifically disclosed in example 3.

Dosage forms may include, but are not limited to: a) pharmaceutical drug delivery systems, including those for oral administration, buccal administration, rectal administration, topical or mucosal delivery, implants for subcutaneous delivery or other implanted drug delivery systems; or b) compositions for delivering minerals, vitamins and other nutraceuticals, oral care agents, flavorants, and the like.

The dosage forms of the present invention are typically considered to be solid; however, they may contain liquid or semi-solid components. Suitable "solid dosage forms" of the present invention include, but are not limited to granules, pellets, multiparticles, tablets, caplets, capsules, lozenges, sachets, dispensable powders and the like.

In a preferred embodiment, the dosage form according to the invention comprises a relative amount of amorphous calcium L-5-methylfolate to cysteine of at least 1:2. Preferably, the relative amount of amorphous calcium L-5-methylfolate to cysteine is of 1:2 to 1:40. More preferably, the relative amount of amorphous calcium L-5-methylfolate to cysteine is of 1:2 to 1:33.

Amorphous L-methylfolate may be present in an amount from about 0.1% to 60%, preferably from about 0.2% to 40%, more preferably from 0.3% to 30% of the total weight of the composition.

Typically, the amount used of amorphous calcium L-5-methylfolate is between 0.1 and 10 mg, preferably 0.4 to 1 mg, particularly preferred 0,451 mg.

Cysteine may be present in an amount from about 0.2% to about 70%, preferably from about 0.5% to 60%, more preferably from about 1% to 50% of the total weight of the composition.

The dosage form of the invention may, if desired, further include one or more auxiliary agents which may be added during the appropriate step so as to afford the appropriate mechanical and release properties. All such auxiliary agents must be compatible with the other ingredients of the dosage form and not injurious to the human being.

The auxiliary agent may be selected from the group consisting of diluents, binders, lubricants, and disintegrating, antiadherent, colouring, sweetening, flavouring agents, and/or mixtures thereof.

Suitably, the dosage form according to the invention comprises a diluent. Diluents are inert excipients that facilitate compression of pulverulent materials and provide resistance to tablets. Suitable diluents include corn starch, microcrystalline cellulose, powdered cellulose, silicified cellulose, lactose monohydrate, anhydrous lactose, mannitol, sorbitol, sucrose, fructose, dextrose, and/or mixtures thereof. Preferably lactose monohydrate and microcrystalline cellulose are used.

Diluents may be presents in an amount from about 30% to about 95%, preferably from 40% to 90%, and more preferably form 50 to 87%.

Suitably, the dosage form according to the invention comprises a binder. Binders are compounds that are able to provide cohesive properties to pulverulent material, in such a way that the fluidity of the composition is improved. The binding agent is selected from the group consisting of hydroxypropyl cellulose, hydroxypropyl methyl cellulose, hydroxyethyl cellulose, methylcellulose, polyvinylpyrrolidone, sodium carboxymethyl cellulose, calcium carboxymethyl cellulose, and/or mixtures thereof. Preferably polyvinylpyrrolidone is used.

Binders may be present in an amount from about 0,5% to about 10% by weight, preferably from about 2% to about 9% by weight, and more preferably from 2.5% to 7.5% by weight of the total weight of the composition.

Disintegrating agents are excipients that promote the rapid breakup of the tablet in an aqueous medium, as well as the rapid disintegration of the granule in order to release more quickly the active substance. Disintegrating agents may be selected from the group consisting of low-substituted hydroxypropyl cellulose, sodium carboxymethyl cellulose, calcium carboxymethyl cellulose, crospovidone, sodium croscarmellose, and/or mixtures thereof. Preferably sodium croscarmellose is used.

Disintegrating agents may be present in an amount from about 1% to about 15% by weight, preferably from about 2% to about 12% by weight, and more preferably from 3% to 10% by weight of the total weight of the composition.

Lubricants and antiadherent agents are excipients that reduce interparticular friction and prevent adhesion of drug particles, and improve fluidity of granular or pulverulent compositions. Lubricants may be selected from the group consisting of talc, alkaline earth salts of stearic acid, specially magnesium and calcium stearate, stearic acid, glycerin palmitostearate, stearyl fumarate, and/or mixtures thereof. Colloidal silica is most preferred as an antiadherent agent.

The lubricant may be present in an amount from about 0% to 5% by weight, preferably from about 0% to about 3% based on the total weight of the composition.

The antiadherent agent may be present in an amount from about 0% to 5% by weight, preferably from about 0% to about 3% based on the total weight of the composition.

The compositions of this invention may also contain sweetening and flavouring agents in order to provide acceptable organoleptic properties (flavour and taste) for patients. Suitable sweetening agents include sodium saccharin, aspartame, mannitol, xylitol, sucrose, sorbitol and ammonium glycyrrhizinate. Suitable flavouring agents include fruit and plant flavours, for example orange, anise, mint, etc. Suitable colouring agents, which may be incorporated into the tablets of the invention, may be selected from those approved for oral use.

The tablets may be coated using conventional methods known to a person skilled in the art, as those described in Remington: The Science and Practice of Pharmacy, 20th Edition, Philadelphia, Lippincott, Williams & Wilkins, 2000 [ISBN 0 683 306472]. Among the film-forming agents which are used for coating the tablets, polyvinylpyrrolidone, hydroxypropyl methyl cellulose, hydroxypropyl cellulose, methyl cellulose, ethyl cellulose, hypromelose, solid polyethylenglycol, and polyvinyl alcohol may be included.

Information on the characteristics of auxiliary agents is described in reference handbooks available to those skilled in the art, for example in Handbook of Pharmaceutical Excipients, 4th Edition, London, Pharmaceutical Press, 2003 [ISBN 0 85369 472 9] wherein, in addition, the trade names of commercially available auxiliary agents are included.

The dosage form of the invention may, if desired, include further active ingredients. In a preferred embodiment, said further active ingredient is selected from the group of estrogens and progestagens. Suitable estrogens are ethinylestradiol, mestranol, quinestranol, estradiol, estrone, estrane, estriol, estetrol and conjugated equine estrogens. Ethinylestradiol is particularly preferred.

Examples of suitable progestogens include levonorgestrel, norgestimate, norethistreone, drospirenone, desogestrel, dienogest cyproterone acetate, chlormadinone, gestodene, progesterone and dihydrogesterone. Drospirenone is particularly preferred.

Preferred amounts of ethinyl estradiol used according to the invention are for example 10 to 50 µg, preferably 10 to 30 µg, and more preferably 20 to 30 µg.

Preferred amounts of drospirenone used according to the invention are for example 0.5 to 5 mg, preferably 3 mg of drospirenone.

In a preferred embodiment, the dosage form according to the invention comprises:
- an effective amount of amorphous calcium L-5-methylfolate,
- cysteine as stabilizing agent
- from 10 to 50 µg of ethynil estradiol
- from 0.5 to 5 mg of drospirenone,
and optionally further auxiliary agents.

In a more preferred embodiment, the dosage form according to the invention comprises:
- from 0.1 and 10 mg of amorphous calcium L-5-methylfolate,
- cysteine as stabilizing agent
- from 10 to 50 µg of ethynil estradiol
- from 0.5 to 5 mg of drospirenone
- and optionally further auxiliary agents,
wherein the relative amount of amorphous calcium L-5-methylfolate to cysteine is of at least 1:2.

Another aspect of the invention is the use of the dosage form of the invention for the production of a pharmaceutical composition, for example, contraceptive composition or for the production of compositions for delivering minerals, vitamins and other nutraceuticals, such as multivitamin preparations.

Still another aspect of the present invention refers to a process for the production of a dosage form comprising amorphous calcium L-5-methylfolate comprising blending amorphous calcium L-5-methylfolate with cysteine and optionally one or more auxiliary agents and then forming the dosage form from the blend.

Dosage forms of the invention may be formed from the blend of amorphous calcium L-5-methylfolate with cysteine using standard techniques, and using standard equipment, known to the skilled person, including direct compression/compaction, wet or dry granulation, spray drying, milling, mixing, drying, encapsulation and coating, as well as combinations of these processes.

In a preferred embodiment, the process of the invention comprises the following steps:
a) blending amorphous calcium L-5-methylfolate with cysteine and optionally one or more pharmaceutically acceptable ingredients, and
b) then forming the dosage form from the blend by:
   i) direct compression of the blend, or
   ii) wet or dry granulation of the blend to form a granulate for incorporation into the dosage form, or
   iii) spray drying the blend to form a multiparticulate for incorporation into the dosage form.

Another aspect of the invention, is the use of a dosage form according to the invention for the production of a pharmaceutical or nutraceutical composition for reducing the risk of a neural tube defect and other congenital malformations caused by folate deficiency, such as cardiac or urogenital defects, cleft lip, jaw and palate.

Another aspect of the invention, is the use of a dosage form according to the invention for the production of a pharmaceutical or nutraceutical composition for the treatment of high risk recurrent pregnancy loss; for reducing the risk of malignant disorders; for reducing the risk of cardiovascular disorders; for reducing the risk of anemia due to impaired folic acid absorption; for the treatment of early memory loss, mild to moderate cognitive impairment and vascular dementia or Alzheimer disease.

The following examples are given only to illustrate the invention in a sufficiently complete manner.

### EXAMPLES

### Example 1. Tablets obtained by direct compression

| **Tablet component** | **(mg/tablet)** | **Function** |
|---|---|---|
| Calcium L-5-methylfolate (amorphous) | 0.45 mg | Active ingredient |
| Cysteine | 4.55 mg | Stabilizing agent |
| Lactose monohydrate | 48.00 mg | Diluent |
| Microcrystalline cellulose | 23.00 mg | Diluent |
| Croscarmellose Sodium | 3.60 mg | Disintegrant |
| Magnesium Stearate | 0.40 mg | Lubricant |
| ***Total*** | ***80. 00 mg*** | |

### Procedure:

a) Accurately weight and mix Calcium L-5-methylfolate (amorphous) and Cysteine for few minutes
b) Mill the resulting mixture using Quadro Comill U3
c) Add Lactose monohydrate, Microcristalline Cellulose and Croscarmellose Sodium to the milled powder obtained in step b. Blend for few minutes.
d) Add Magnesium Stearate
e) The final blend is then compressed using a rotary tablet machine Kilian S100 MX with punches of diameter 6 mm at the weight of 80 mg.

### Example 2. Tablets obtained by granulation

| **Tablet component** | **(mg/tablet)** | **Function** |
|---|---|---|
| Calcium L-5-methylfolate (amorphous) | 0.90 mg | Active ingredient |
| Cysteine | 6.10 mg | Stabilizing agent |
| Lactose Monohydrate | 48.00 mg | Diluent |
| Povidone K30 | 4.00 mg | Binder |
| Starch | 17.00 mg | Diluent |
| Croscarmellose Sodium | 3.6 mg | Disintegrant |
| Magnesium Stearate | 0.40 mg | Lubricant |
| *Ethanol** | *(20.00 mg)* | Granulation solvent |
| ***Total*** | ***80.00 mg*** | |

| | | |
|---|---|---|
| * evaporated during the process | | |

### Procedure:

a) Calcium L-5-methylfolate (amorphous) and Cysteine are accurately weighted and blended for few minutes
b) Lactose monohydrate is then added to the blend obtained in step a
c) A binder solution containing PVP dissolved in ethanol is added to the blend obtained in step b.
   When all the binder solution has been added, the granules are dried at 60°C under vacuum.
d) Croscarmellose sodium and magnesium stearate are then added to the granules obtained in step c
e) The final blend is then compressed into tablets using a rotary tablet machine Kilian S100 MX with punches of diameter 6 mm at the weight of 80 mg.

### Example 3. Tablets obtained by spray drying

| **Tablet component** | **(mg/tablet)** | **Function** |
|---|---|---|
| Calcium L-5-methylfolate (amorphous) | 1.80 mg | Active Ingredient |
| Cysteine | 8.20 mg | Stabilizing agent |
| Lactose (Spray-Dried) | 31.00 mg | Diluent |
| Microcrystalline Cellulose | 31.00 mg | Diluent |
| Aerosil 200 | 0.40 mg | Antiadherent agent |
| Croscarmellose Sodium | 7.20 mg | Disintegrant |
| Magnesium Stearate | 0.40 mg | Lubricant |
| *Purified water** | *(100 mg)* | Solvent |
| ***Total*** | ***80.00 mg*** | |

| | | |
|---|---|---|
| * evaporated during the process | | |

a) Calcium L-5-methylfolate (amorphous) and Cysteine are dissolved in water with gentle stirring to obtain a clear solution.
b) The solution is then spray-dried with constant stirring using a Büchi Mini Spray Dryer B-290 (Büchi laboratory-Techniques, Switzerland) using the following conditions:
   a. spraying air flow, 600 L/h;
   b. drying air flow, 35 m³/h;
   c. suspension feed rate, 4.0 g/min;
   d. nozzle size, 0.7 mm.
   The inlet temperature was established at 130°C and, in these conditions, the outlet temperature was about 70°C.
c) The resultant powder is then blended with Spray-Dried Lactose, Microcrystalline Cellulose, Aerosil 200, Croscarmellose Sodium and Magnesium Stearate.
d) The powder is then passed powder through 0.8 mm steel sieve and transfer in a cubic mixer; then the blend is mixed for 10 minutes at 10 r.p.m.
e) The mixture obtained in step d is compressed using a rotary tablet machine Kilian S100 MX with punches of diameter 6 mm at the weight of 80 mg.

### Example 4. Tablets obtained by spray drying

| **Tablet component** | **(mg/tablet)** | **Function** |
|---|---|---|
| Calcium L-5-methylfolate (amorphous) | 1.80 mg | Active ingredient |
| Cysteine | 8.20 mg | Stabilizing agent |
| Lactose monohydrate | 8.00 mg | Diluent |
| Microcrystalline Cellulose | 55.00 mg | Diluent |
| Aerosil 200 | 0.40 mg | Antiadherent |
| Croscarmellose Sodium | 6.20 mg | Disintegrant |
| Magnesium Stearate | 0.40 mg | Lubricant |
| *Purify water** | *(150 mg)* | Solvent |
| ***Total*** | ***80.00 mg*** | |

| | | |
|---|---|---|
| * evaporated during the process | | |

### Procedure:

a) Calcium L-5-methylfolate (amorphous), Cysteine and Lactose monohydrate are dissolved in water with gentle stirring to obtain a clear solution.
b) The solution is then spray-dried with constant stirring using a Büchi Mini Spray Dryer B-290 (Büchi laboratory-Techniques, Switzerland) using the following conditions:
   a. spraying air flow, 600 L/h;
   b. drying air flow, 35 m³/h;
   c. suspension feed rate, 4.0 g/min;
   d. nozzle size, 0.7 mm.
      The inlet temperature is established at 130°C and, in these conditions, the outlet temperature is about 70°C.
c) The resultant powder is then blended with Microcrystalline Cellulose, Aerosil 200, Croscarmellose Sodium and Magnesium Stearate.
d) The powder is then passed powder through 0.8 mm steel sieve and transfer in a cubic mixer; then the blend is mixed for 10 minutes at 10 r.p.m.
e) The mixture obtained in step d is compressed using a rotary tablet machine Kilian S100 MX with punches of diameter 6 mm at the weight of 80 mg.

### Example 5. Contraceptive formulation comprising Calcium L-5-methylfolate (amorphous)

| **Tablet component** | **(mg/tablet)** | **Function** |
|---|---|---|
| Drospirenone | 3.000 mg | Active Ingredient |
| Ethynil Estradiol | 0.020 mg | Active Ingredient |
| Calcium L-5-methylfolate (amorphous) | 0.451 mg | Active Ingredient |
| Cysteine | 5.000 mg | Stabilizing agent |
| Lactose monohydrate | 48.529 mg | Diluent |
| Microcrystalline cellulose | 20.000 mg | Diluent |
| Croscarmellose Sodium | 2.500 mg | Disintegrant |
| Magnesium Stearate | 0.500 mg | Lubricant |
| ***Total*** | ***80.00 mg*** | |

### Procedure:

a) Calcium L-5-methylfolate (amorphous) and Cysteine are accurately weighted and blended for few minutes
b) The mixture obtained is then milled using Quadro Comill u3
c) Drospirenone, Ethynil estradiol, Lactose monohydrate, Microcristalline Cellulose and Croscaramellose Sodium are added to the milled powder obtained in step b and then blended for few minutes.
d) Magnesium Stearate is added to the mixture obtained in step c
e) The mixture obtained in step d is compressed using a rotary tablet machine Kilian S100 MX with punches of diameter 6 mm at the weight of 80 mg.

### Example 6. Amorphous Calcium L-5-methylfolate stability texts.

Powder mixtures of amorphous calcium L-5-methylfolate with each of the stabilizing agents disclosed in the following table were prepared. In all the cases, the mixture contained an amount of calcium L-5-methylfolate to stabilizing agent of 1:10. Table 1 shows the amount of Calcium L-5-methylfolate that remains in the mixture after storage at 5°C for one month.

**Table 1**

| **Sample** | **Assay (%)** | **Related substances (%)** | | | |
|---|---|---|---|---|---|
| | | **HO-MeTHFA¹** | **D-Mefox²** | **L-Mefox³** | **Total impurities** |
| Amorphous Calcium L-5-methylfolate (ALC) | 91.02 | 1.18 | 0.50 | 4.27 | 8.0 |
| ALC + alpha-tocopherol | 57.04* | 0.14 | 0.28 | 3.47 | 5.6 |
| ALC + Potassium Polacrilin | 91.95 | 1.57 | 0.23 | 4.20 | 8.6 |
| ALC + (BHA) | 77.69 | 1.57 | 0.60 | 5.09 | 8.8 |
| ALC + (BHT) | 78.50 | 1.33 | 0.52 | 4.52 | 8.6 |
| ALC + Lipoic Acid | 87.91 | 0.35 | 0.61 | 3.86 | 6.9 |
| ALC + Ascorbyl palmitate | 86.03 | 0.35 | 0.42 | 4.14 | 6.9 |
| ALC + Citric acid | 89.75 | - | 0.45 | 4.05 | 6.9 |
| ALC + Tri-sodium Dihydrated Citrate | 84.93 | 3.19 | 0.25 | 5.29 | 11.7 |
| **ALC + Cysteine** | **102.10** | - | - | **0.81** | **1.7** |
| ALC + Propyl Galate | 95.39 | 1.80 | 0.54 | 4.79 | 9.6 |
| ALC + Sodium ascorbate | 92.66 | 4.01 | - | 4.01 | 10.8 |
| ALC + Calcium ascorbate | 91.76 | 0.80 | - | 4.00 | 7.9 |
| ALC + BHA + BHT | 97.03 | 1.62 | 0.41 | 5.06 | 9.2 |
| ALC + BHA + BHT + Citric acid | 87.90 | - | 0.36 | 3.85 | 6.2 |
| ALC + Ascorbil palmitate + alpha-tocopherol | 56.70* | - | 0.25 | 3.46 | 6.5 |
| ALC + Ascorbil palmitate + alpha-tocopherol + Citric acid | 43.95* | - | 0.48 | 3.27 | 6.5 |

| | | | | | |
|---|---|---|---|---|---|
| ^{1.} HO-MeTHFA: 4α-hydroxy-5-methyltetrahydrofolic acid ^{2.} D-Mefox: D-pyrazino-S-triazine derivative ^{3.} L-Mefox: L- pyrazino-S-triazine derivative | | | | | |

The results show that the cysteine is the only agent able to stop the degradation of Amorphous Calcium L-5-methylfolate.

In order to confirm the stabilization effect of cysteine on amorphous Calcium L-5-methylfolate, additional test were conducted with both substances, at ratios of from 1:0 to 1:33.

**Table 2. Amorphous Calcium L-5-methylfolate (ALC) assay and related substances found after storage of ALC and cysteine at different ratios at 5°C for 1 month.**

| **Sample** | **Ratio ALC: Cysteine** | **Assay (%)** | **Related substances (%)** | | | |
|---|---|---|---|---|---|---|
| | | | **HO-MeTHFA** | **D-Mefox** | **L-Mefox** | **TOTAL impurities** |
| A | 1:0 | 99.59 | 1.80 | 0.42 | 2.52 | 5.59 |
| B | 1:11 | 101.56 | - | - | 1.72 | 2.13 |
| C | 1:22 | 100.14 | - | - | 1.58 | 1.58 |
| D | 1:33 | 100.76 | - | - | 1.61 | 1.90 |

**Table 3. Amorphous Calcium L-5-methylfolate (ALC) assay and related substances found after storage of ALC and cysteine at different ratios at 5°C for 3 months.**

| **Sample** | **Ratio ALC: Cysteine** | **Assay (%)** | **Related substances (%)** | | | |
|---|---|---|---|---|---|---|
| | | | **HO-MeTHFA** | **D-Mefox** | **L-Mefox** | **TOTAL impurities** |
| A | 1:0 | 96.39 | 1.34 | 0.32 | 2.74 | 5.74 |
| B | 1:11 | 99.63 | - | - | 1.66 | 1.66 |
| C | :22 | 99.14 | - | - | 1.59 | 1.59 |
| D | 1:33 | 103.36 | - | - | 1.58 | 1.58 |

**Table 4. Amorphous Calcium L-5-methylfolate (ALC) assay and related substances found after storage of ALC and cysteine at different ratios at 25°C/60% HR for 1 month.**

| **Sample** | **Ratio ALC: Cysteine** | **Assay (%)** | **Related substances (%)** | | | |
|---|---|---|---|---|---|---|
| | | | **HO-MeTHFA** | **D-Mefox** | **L-Mefox** | **TOTAL impurities** |
| A | 1:0 | 89.13 | 1.75 | 0.71 | 4.68 | 9.19 |
| B | 1:11 | 97.01 | - | - | 1.59 | 1.59 |
| C | 1:22 | 98.26 | - | - | 1.63 | 2.26 |
| D | 1:33 | 98.07 | - | - | 1.52 | 2.14 |

**Table 5. Amorphous Calcium L-5-methylfolate (ALC) assay and related substances found after storage of ALC and cysteine at different ratios at 25°C/60% HR for 3 months.**

| **Sample** | **Ratio ALC: Cysteine** | **Assay (%)** | **Related substances (%)** | | | |
|---|---|---|---|---|---|---|
| | | | **HO-MeTHFA** | **D-Mefox** | **L-Mefox** | **TOTAL impurities** |
| A | 1:0 | 84.03 | 1.44 | 0.57 | 6.49 | 11.95 |
| B | 1:11 | 97.92 | - | - | 1.65 | 3.06 |
| C | 1:22 | 98.42 | - | - | 1.50 | 2.91 |
| D | 1:33 | 98.43 | - | - | 1.48 | 2.84 |

**Table 6. Amorphous Calcium L-5-methylfolate (ALC) assay and related substances found after storage of LC and cysteine at different ratios at 40°C/75% HR for 1 month.**

| **Sample** | **Ratio ALC: Cysteine** | **Assay (%)** | **Related substances (%)** | | | | |
|---|---|---|---|---|---|---|---|
| | | | **HO-MeTHFA** | **D-Mefox** | **L-Mefox** | **THFA** | **TOTAL impurities** |
| A | 1:0 | 81.95 | 1.84 | 0.93 | 8.97 | - | 15.57 |
| B | 1:11 | 88.64 | - | - | 1.21 | 0.40 | 8.33 |
| C | 1:22 | 90.90 | - | - | 1.26 | 0.51 | 8.28 |
| D | 1:33 | 90.83 | - | - | 1.12 | 0.37 | 8.17 |

The results show that the Cysteine is able to improve the stability of amorphous Calcium L-5-methylfolate. The impurities amount found without cysteine is significantly higher than when cysteine is not used. No significant differences are observed between the tested ratios for each condition.

The most significant degradation was found at the most aggressive storage conditions (40°C/75% HR). However, even in this case, the degradation of the LC was reduced when cysteine was used, for all the ratios.

The results obtained at the other tested conditions show also that cysteine is able to stabilize amorphous Calcium L-5-methylfolate during the time: no significant increase of the impurities or decrease on the assay value was found between storage at 1 month or 3 months at 25°C/60% HR when cysteine was used.

In case of storage at 5°C, the effect of the cysteine over the time is less important, however it is important to note that the impurities amount is significantly higher than when cysteine is used.

## Claims

1. A dosage form comprising an effective amount of amorphous calcium L-5-methylfolate as active ingredient, cysteine as stabilizing agent and optionally further auxiliary agents.

2. A dosage form according to claim 1, wherein the relative amount of amorphous calcium L-5-methylfolate to cysteine is at least 1:2.

3. A dosage form according to claims 1 and 2, wherein the relative amount of amorphous calcium L-5-methylfolate to cysteine is 1:2 to 1:33.

4. A dosage form according to claims 1 to 3, further comprising:
- from 0.5 to 5 mg of drospirenone

5. A dosage form according to claims 1 to 3, further comprising:
- from 10 to 50 µg of ethynil estradiol
- from 0.5 to 5 mg of drospirenone

6. A dosage form according to claims 1 to 3, further comprising:
- from 0.1 and 10 mg of amorphous calcium L-5-methylfolate,
- from 10 to 50 µg of ethynil estradiol
- from 0.5 to 5 mg of drospirenone

7. A process for the production of a dosage form according to claims 1 to 6, comprising blending amorphous calcium L-5-methylfolate with cysteine and optionally one or more auxiliary agents and then forming the dosage form from the blend.

8. A process according to claim 7, comprising the following steps:
a) blending amorphous calcium L-5-methylfolate with cysteine and optionally one or more pharmaceutically acceptable ingredients, and
b) then forming the dosage form from the blend by:
i) direct compression of the blend, or
ii) wet or dry granulation of the blend to form a granulate for incorporation into the dosage form, or
iii) spray drying the blend to form a multiparticulate for incorporation into the dosage form.

9. Use of a dosage form according to claims 1 to 6 for the production of a pharmaceutical composition.

10. Use of a dosage form according to claims 1 to 6 for the production of nutraceuticals composition.

11. Use of a dosage form according to claims 1 to 6 for the production of a pharmaceutical or nutraceutical composition for reducing the risk of a neural tube defect and other congenital malformations caused by folate deficiency.

12. Use according to claim 11, wherein congenital malformations caused by folate deficiency are cardiac or urogenital defects, cleft lip, jaw and palate.

13. Use of a dosage form according to claims 1 to 6 for the production of a pharmaceutical or nutraceutical composition for the treatment of high risk recurrent pregnancy loss; for reducing the risk of malignant disorders; for reducing the risk of cardiovascular disorders; for reducing the risk of anemia due to impaired folic acid absorption; for the treatment of early memory loss, mild to moderate cognitive impairment and vascular dementia or Alzheimer disease.

14. Use of cysteine for the stabilization of amorphous L-5-methylfolate.

15. Use of cysteine for the stabilization of a dosage form comprising amorphous L-5-methylfolate.
